# EUROPEAN PATENT APPLICATION

(11) **EP 4 751 758 A1**
(43) Date of publication of application: **03.06.2026**
(21) Application number: 24854210.2
(22) Date of filing: 13.08.2024
(51) Int. Cl.: A61M 39/10, A61M 39/26

(54) **MALE CONNECTOR**

(30) Priority: 17.08.2023 JP 2023133024
(71) Applicant: Terumo Kabushiki Kaisha, Tokyo 151-0072 (JP)
(72) Inventor: AKIYAMA Kazuya, Nakakoma-gun, Yamanashi 409-3853 (JP)
(74) Representative: KIPA AB
(86) International application number: PCT/JP2024/028933
(87) International publication number: WO 2025/037621

(57) **Abstract**

A male connector connectable to a female connector having an elastic valve body includes: a flow path pipe member having an opening; an elastic opening and closing portion; a biasing portion; a housing; and a movable body that includes a movable body restriction portion and holds the elastic opening and closing portion. A form is changed from a first form in which the opening is closed by the elastic opening and closing portion to a second form in which the opening is opened by the flow path pipe member protruding from the elastic opening and closing portion by a connection operation in which the elastic opening and closing portion and the movable body move to an axial proximal side against a biasing force by the biasing portion by pressing the elastic valve body against the elastic opening and closing portion. The movable body restriction portion restricts movement of the female connector with respect to the elastic opening and closing portion by moving inward in a radial direction. The housing includes a housing restriction portion that restricts the movement of the female connector through the movable body restriction portion by restricting movement of the movable body according to a change from the first form to the second form.

## Description

### Technical Field

The present disclosure relates to a male connector.

### Background Art

There is known a male connector connectable to a female connector having an elastic valve body (for example, see PTL 1). The male connector includes: a flow path pipe member having an opening; an elastic opening and closing portion; a biasing portion; a housing; and a movable body that includes a movable body restriction portion and holds the elastic opening and closing portion. A form is changed from a first form in which the opening is closed by the elastic opening and closing portion to a second form in which the opening is opened by the flow path pipe member protruding from the elastic opening and closing portion by a connection operation in which the elastic opening and closing portion and the movable body move to an axial proximal side against a biasing force by the biasing portion by pressing the elastic valve body against the elastic opening and closing portion. The movable body restriction portion restricts movement of the female connector with respect to the elastic opening and closing portion by moving inward in a radial direction. The housing includes a housing restriction portion that restricts the movement of the female connector according to a change from the first form to the second form.

### Citation List

### Patent Literature

PTL 1: WO2022/149339

### Summary of Invention

### Technical Problem

It is preferable that the male connector as described above can prevent leakage of a fluid when the male connector is connected to a female connector.

Therefore, an object of the disclosure is to provide a male connector capable of preventing leakage of a fluid when the male connector is connected to a female connector.

### Solution to Problem

One aspect of the disclosure is as follows.
[1] A male connector connectable to a female connector having an elastic valve body, the male connector comprising:
   a flow path pipe member that defines an axial direction and a radial direction and has an opening at a portion on an axial distal side;
   an elastic opening and closing portion;
   a biasing portion capable of biasing the elastic opening and closing portion toward the axial distal side;
   a housing; and
   a movable body that includes a movable body restriction portion and holds the elastic opening and closing portion, wherein
   a form is changed from a first form in which the opening of the flow path pipe member is closed by the elastic opening and closing portion to a second form in which the opening of the flow path pipe member is opened by a portion of the flow path pipe member on the axial distal side protruding from the elastic opening and closing portion to the axial distal side by a connection operation in which the elastic opening and closing portion and the movable body move to an axial proximal side with respect to the flow path pipe member against a biasing force by the biasing portion by pressing the elastic valve body against a distal end surface of the elastic opening and closing portion,
   the movable body restriction portion restricts movement of the female connector to the axial distal side with respect to the elastic opening and closing portion by moving inward in the radial direction due to a resistance force from the housing generated according to the connection operation, and
   the housing includes a housing restriction portion that restricts the movement of the female connector to the axial distal side with respect to the flow path pipe member through the movable body restriction portion by restricting movement of the movable body to the axial distal side with respect to the flow path pipe member according to a change from the first form to the second form.
[2] The male connector according to [1], in which
   the movable body restriction portion restricts the movement of the female connector to the axial distal side with respect to the elastic opening and closing portion by being hooked on the female connector.
[3] The male connector according to [1] or [2], in which
   the housing restriction portion restricts the movement of the movable body to the axial distal side by being hooked on the movable body or the elastic opening and closing portion.
[4] The male connector according to any one of [1] to [3], in which
   the movable body includes a housing hooked portion on the axial proximal side with respect to the movable body restriction portion, and
   the housing restriction portion includes a housing hooking portion to be hooked on the housing hooked portion.
[5] The male connector according to any one of [1] to [4], in which
   the movable body restriction portion includes an arm extending radially outward and toward the axial distal side, and a protrusion protruding inward in the radial direction from an end portion of the arm on the axial distal side.
[6] The male connector according to [5], in which
   the protrusion of the movable body restriction portion is located on the axial distal side with respect to the distal end surface of the elastic opening and closing portion in the first form.
[7] The male connector according to any one of [1] to [6], in which
   the movable body includes a plurality of the movable body restriction portions arranged in a circumferential direction defined by the flow path pipe member.
[8] The male connector according to [7], in which
   the movable body includes a pair of the movable body restriction portions facing each other in a predetermined radial direction and having an equal width in the circumferential direction, and another pair of the movable body restriction portions facing each other in another radial direction perpendicular to the predetermined radial direction and having an equal width in the circumferential direction.
[9] The male connector according to any one of [1] to [8], in which
   the elastic opening and closing portion and the biasing portion form an integrated elastic member.
[10] The male connector according to any one of [1] to [9], in which
   the male connector is configured as a medical instrument.
[11] A connection structure including:
   the male connector according to any one of [1] to [10]; and
   the female connector.
[12] The connection structure according to [11], in which
   the male connector and the female connector are each configured as a medical instrument.

### Advantageous Effect of Invention

According to the disclosure, it is possible to provide a male connector capable of preventing leakage of a fluid when the male connector is connected to a female connector.

### Brief Description of Drawings

[Fig. 1] Fig. 1 is a cross-sectional view showing a state before a male connector is connected to a female connector in a first embodiment of the disclosure.
[Fig. 2] Fig. 2 is a cross-sectional view at an angle different from that in Fig. 1 by 90°.
[Fig. 3] Fig. 3 is a cross-sectional view showing a state when a connection operation is started from the state shown in Fig. 1.
[Fig. 4] Fig. 4 is a cross-sectional view at an angle different from that in Fig. 3 by 90°.
[Fig. 5] Fig. 5 is a cross-sectional view showing a state when the connection operation is completed from the state shown in Fig. 3.
[Fig. 6] Fig. 6 is a cross-sectional view showing a state when a connection operation between a male connector and a female connector is completed in a second embodiment of the disclosure.

### Description of Embodiments

Hereinafter, embodiments of the disclosure will be exemplarily described in detail with reference to the drawings.

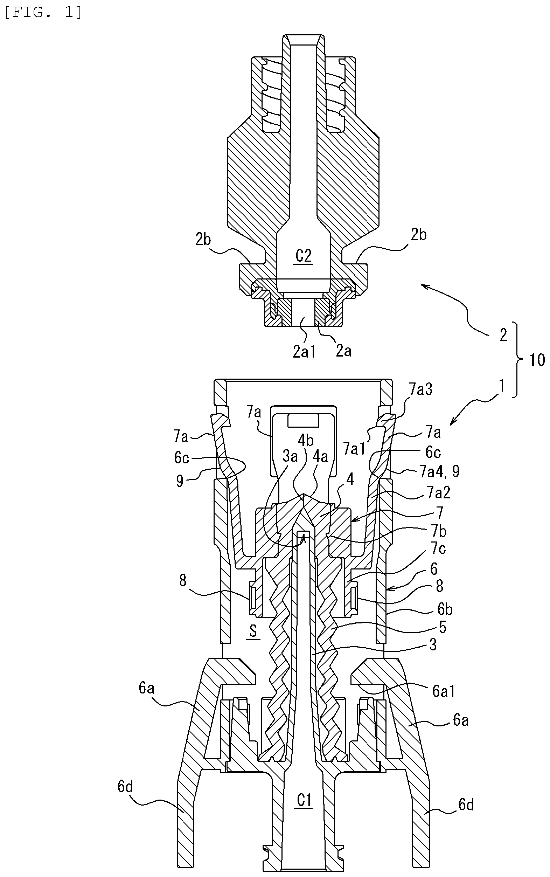

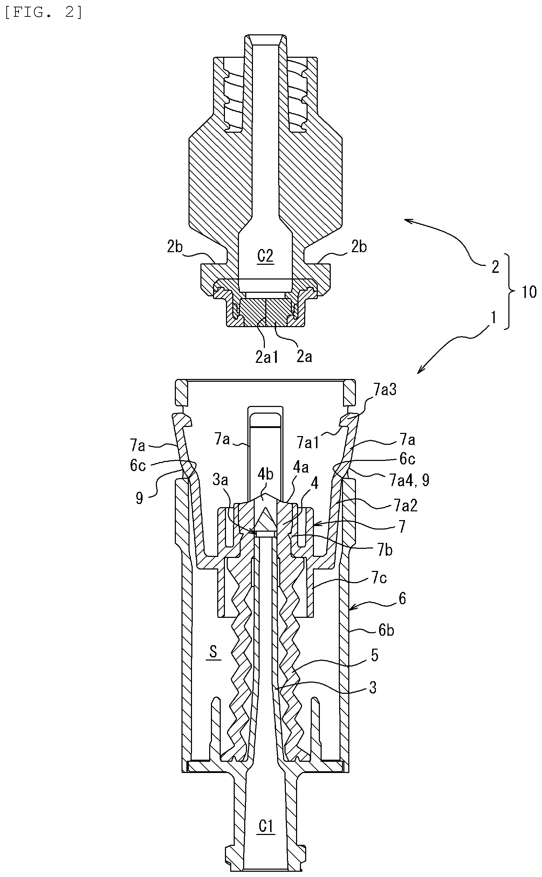

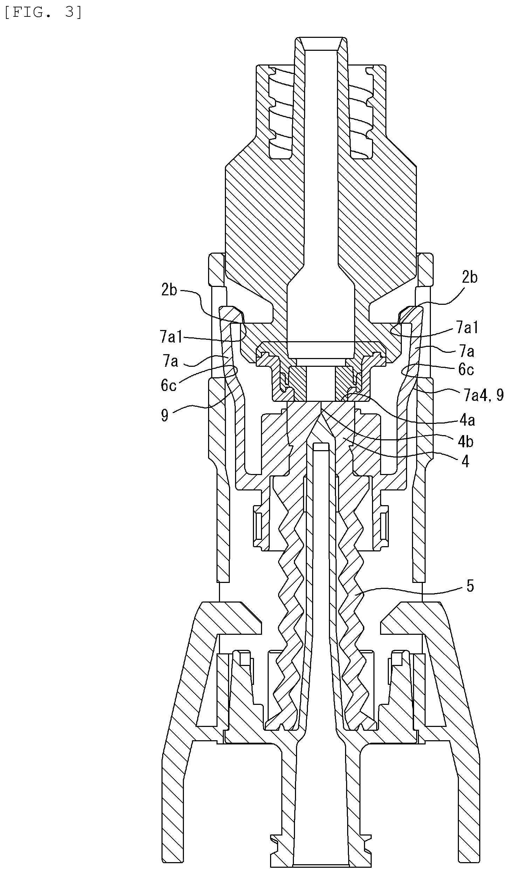

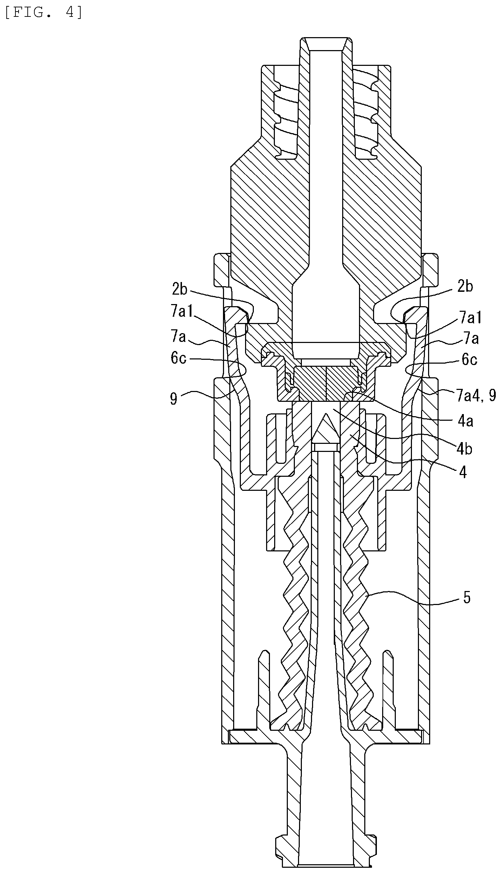

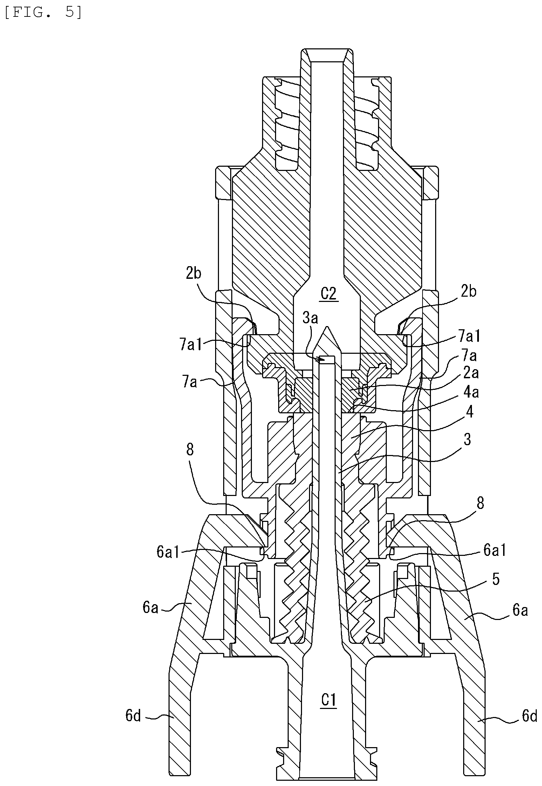

As shown in Figs. 1 to 5, in a first embodiment of the disclosure, a male connector 1 connectable to a female connector 2 having an elastic valve body 2a includes: a flow path pipe member 3 that defines an axial direction and a radial direction and has an opening 3a at a portion on an axial distal side; an elastic opening and closing portion 4; a biasing portion 5 capable of biasing the elastic opening and closing portion 4 toward the axial distal side; a housing 6; and a movable body 7 that includes a movable body restriction portion 7a and holds the elastic opening and closing portion 4. A form is changed from a first form in which the opening 3a of the flow path pipe member 3 is closed by the elastic opening and closing portion 4 to a second form in which the opening 3a of the flow path pipe member 3 is opened by a portion of the flow path pipe member 3 on the axial distal side protruding from the elastic opening and closing portion 4 to the axial distal side by a connection operation in which the elastic opening and closing portion 4 and the movable body 7 move to an axial proximal side with respect to the flow path pipe member 3 against a biasing force by the biasing portion 5 by pressing the elastic valve body 2a against a distal end surface 4a of the elastic opening and closing portion 4. The movable body restriction portion 7a restricts movement of the female connector 2 to the axial distal side with respect to the elastic opening and closing portion 4 by moving inward in the radial direction due to a resistance force from the housing 6 generated according to the connection operation. The housing 6 includes a housing restriction portion 6a that restricts the movement of the female connector 2 to the axial distal side with respect to the flow path pipe member 3 through the movable body restriction portion 7a by restricting movement of the movable body 7 to the axial distal side with respect to the flow path pipe member 3 according to a change from the first form to the second form.

According to the above configuration, by restricting the movement of the female connector 2 to the axial distal side with respect to the elastic opening and closing portion 4 by the movable body restriction portion 7a according to the connection operation, the elastic valve body 2a can be favorably brought into close contact with the elastic opening and closing portion 4. In the second form in a close contact state, a male connector flow path C1 in the flow path pipe member 3 is communicated with a female connector flow path C2 in the female connector 2 through the opening 3a, and the movement of the female connector 2 to the axial distal side with respect to the flow path pipe member 3 is restricted by the housing restriction portion 6a, so that the second form can be maintained. When the connection between the female connector 2 and the male connector 1 is released from the second form in which the connection between the female connector 2 and the male connector 1 is completed and the opening 3a of the flow path pipe member 3 is opened, for example, by performing an operation of pulling out the female connector 2, even in a case where the female connector 2 moves to the distal side at a speed at which a return operation of the elastic opening and closing portion 4 by the biasing portion 5 does not catch up, the movable body 7 can follow the female connector 2 together with the elastic opening and closing portion 4 by the movable body restriction portion 7a while the elastic valve body 2a is favorably brought into close contact with the elastic opening and closing portion 4 until the first form in which the opening 3a of the flow path pipe member 3 is closed is obtained, and thus it is possible to prevent leakage of a fluid. Further, according to the above configuration, when the form is changed from the first form to the second form, since the housing restriction portion 6a restricts the movement of the female connector 2 through the movable body restriction portion 7a without directly restricting the movement of the female connector 2, compared to a case where the female connector 2 is directly restricted, it is possible to secure a wide space for providing a structure for restriction by the movable body restriction portion 7a. As a result, it is possible to improve adhesion between the elastic opening and closing portion 4 and the elastic valve body 2a at the time of connection and disconnection. Therefore, it is possible to implement the male connector 1 capable of preventing the leakage of the fluid when the male connector 1 is connected to the female connector 2.

The movable body restriction portion 7a restricts the movement of the female connector 2 to the axial distal side with respect to the elastic opening and closing portion 4 by being hooked on the female connector 2. That is, the movable body restriction portion 7a includes a movable body hooking portion 7a1 that is hooked on a movable body hooked portion 2b of the female connector 2. According to the above configuration, the movable body restriction portion 7a can be implemented with a simple structure.

The housing restriction portion 6a restricts the movement of the movable body 7 to the axial distal side by being hooked on the movable body 7. According to the above configuration, the movable body 7 and the housing restriction portion 6a can be implemented with simple structures.

The movable body 7 includes a housing hooked portion 8 on the axial proximal side with respect to the movable body restriction portion 7a. The housing restriction portion 6a includes a housing hooking portion 6a1 to be hooked on the housing hooked portion 8. According to the above configuration, the movable body 7 and the housing restriction portion 6a can be further implemented with simpler structures.

The housing restriction portion 6a is hooked on the movable body 7 by swinging. According to the above configuration, hooking by the housing restriction portion 6a can be implemented with a simple structure.

The housing restriction portion 6a is hooked on the movable body 7 by swinging inward in the radial direction of the flow path pipe member 3 due to restoration from elastic deformation. According to the above configuration, hooking by swinging can be implemented with a simple structure.

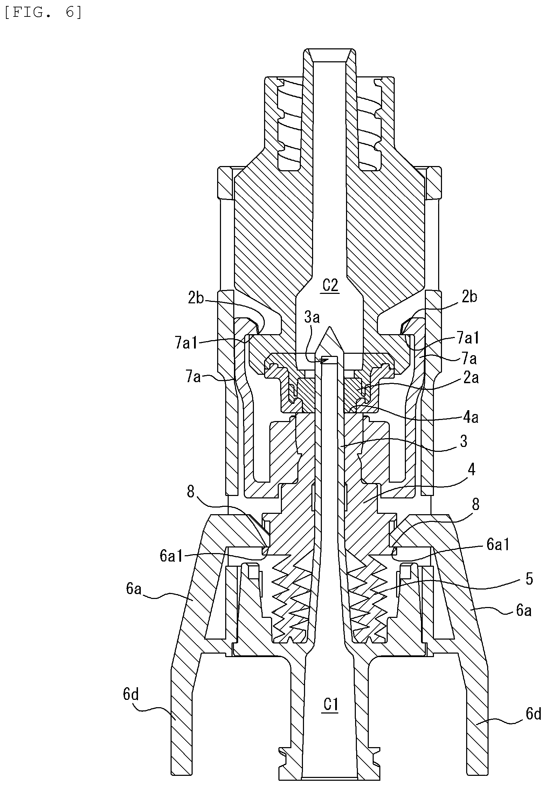

As in a second embodiment shown in Fig. 6, the housing restriction portion 6a may be configured to be hooked on the elastic opening and closing portion 4 instead of being configured to be hooked on the movable body 7. With the above configuration, the movable body 7 and the housing restriction portion 6a can also be implemented with simple structures.

The movable body restriction portion 7a includes an arm 7a2 extending radially outward and toward the axial distal side, and a protrusion 7a3 protruding inward in the radial direction from an end portion of the arm 7a2 on the axial distal side. According to the above configuration, hooking by the movable body restriction portion 7a can be implemented with a simple structure.

The protrusion 7a3 of the movable body restriction portion 7a is located on the axial distal side with respect to the distal end surface 4a of the elastic opening and closing portion 4 in the first form. According to the above configuration, it is possible to implement the movable body 7, which includes the movable body restriction portion 7a hooked on the female connector 2, with a simple structure.

The movable body 7 includes a plurality of the movable body restriction portions 7a arranged in a circumferential direction defined by the flow path pipe member 3. According to the above configuration, it is possible to implement a favorable restriction of the movement of the female connector 2 by the movable body restriction portion 7a. From this viewpoint, the movable body 7 preferably includes the three or more movable body restriction portions 7a arranged in the circumferential direction.

The movable body 7 includes a pair of the movable body restriction portions 7a facing each other in a predetermined radial direction and having an equal width in the circumferential direction, and another pair of the movable body restriction portions 7a facing each other in another radial direction perpendicular to the predetermined radial direction and having an equal width in the circumferential direction. That is, the movable body 7 includes the four movable body restriction portions 7a at positions shifted by 90°. According to the above configuration, it is possible to implement a further favorable restriction of the movement of the female connector 2 by the movable body restriction portion 7a.

By the connection operation in which the elastic valve body 2a is pressed against the elastic opening and closing portion 4 and the elastic opening and closing portion 4 and the movable body 7 move to the axial proximal side with respect to the flow path pipe member 3 against the biasing force of the biasing portion 5, the movable body restriction portion 7a swings inward in the radial direction of the flow path pipe member 3 to a position at which the movable body restriction portion 7a can be hooked on the female connector 2 by the resistance force inward in the radial direction from the housing 6 (a housing base portion 6b). According to the above configuration, hooking by the movable body restriction portion 7a can be implemented with a simple structure.

Therefore, the movable body restriction portion 7a may include a guided portion 7a4 that is guided by a guide portion 6c of the housing 6 (housing base portion 6b) according to the connection operation. At least one of the guide portion 6c and the guided portion 7a4 can include an inclined portion 9 that is inclined radially inward from the distal side to the proximal side. According to the above configuration, as shown in Figs. 1 to 4, since the guided portion 7a4 can be guided radially inward by the guide portion 6c during the connection operation, the movable body restriction portion 7a can be swung radially inward. In the embodiment, the guided portion 7a4 includes the inclined portion 9.

The movable body 7 includes a holding portion 7b that holds the elastic valve body 2a, the movable body restriction portion 7a, and a movable body base portion 7c that extends from the holding portion 7b to the axial proximal side and that connects the holding portion 7b and a root of the movable body restriction portion 7a. The movable body base portion 7c includes the housing hooked portion 8. According to the above configuration, the movable body 7 can be further implemented with a simpler structure.

The movable body restriction portion 7a, the holding portion 7b, and the movable body base portion 7c form the integrated movable body 7. According to the above configuration, the movable body 7 can be formed by a simple manufacturing method such as injection molding.

The housing 6 includes the housing base portion 6b that connects the flow path pipe member 3 and the housing restriction portion 6a and that forms a hollow S where the flow path pipe member 3 is located. The housing base portion 6b includes the guide portion 6c. According to the above configuration, the housing 6 can be implemented with a simple structure.

The housing 6 includes an operation unit 6d extending from the housing restriction portion 6a to the proximal side. The housing restriction portion 6a can be released from being hooked on the movable body 7 by being swung outward in the radial direction of the flow path pipe member 3 by elastic deformation by an operation on the operation unit 6d inward in the radial direction. According to the above configuration, hooking can be released by swinging with a simple structure.

The housing 6 includes a first housing restriction portion 6a and a second housing restriction portion 6a. The flow path pipe member 3 is located between the first housing restriction portion 6a and the second housing restriction portion 6a, or the first housing restriction portion 6a and the second housing restriction portion 6a are provided (to be shifted by 180°) to face each other. According to the above configuration, the second form can be favorably maintained with a simple structure.

The housing 6 includes a first operation unit 6d extending from the first housing restriction portion 6a to the proximal side, and a second operation unit 6d extending from the second housing restriction portion 6a to the proximal side. According to the above configuration, a favorable operation can be implemented with a simple structure.

The flow path pipe member 3 and a root portion of the housing base portion 6b form an integrated member. According to the above configuration, the member can be formed by a simple manufacturing method such as injection molding. The housing restriction portion 6a, a portion of the housing base portion 6b other than the root portion, and the operation unit 6d form an integrated member. According to the above configuration, the member can be formed by a simple manufacturing method such as injection molding.

The elastic opening and closing portion 4 and the biasing portion 5 form an integrated elastic member. According to the above configuration, the male connector 1 can be implemented with a simple structure.

The elastic opening and closing portion 4 has a slit 4b through which the flow path pipe member 3 passes when the form changes from the first form to the second form. According to the above configuration, the elastic opening and closing portion 4 can be implemented with a simple structure.

The elastic opening and closing portion 4 and the biasing portion 5 are formed of, for example, rubber or elastomer. The movable body 7, the flow path pipe member 3, and the housing 6 are formed of, for example, resin.

The male connector 1 is configured as a medical instrument. According to the above configuration, it is possible to implement the male connector 1 as a medical instrument capable of preventing leakage of a fluid when connected to the female connector 2.

A connection structure 10 according to the embodiment includes the male connector 1 and the female connector 2. According to the above configuration, it is possible to implement the connection structure 10 capable of preventing leakage of a fluid when the male connector 1 is connected to the female connector 2.

The elastic valve body 2a has a slit 2a1 through which the flow path pipe member 3 passes when the form changes from the first form to the second form. According to the above configuration, the elastic valve body 2a can be implemented with a simple structure. The elastic valve body 2a is formed of, for example, rubber or elastomer. A portion of the female connector 2 other than the elastic valve body 2a is formed of, for example, resin.

The male connector 1 and the female connector 2 are each configured as a medical instrument. According to the above configuration, it is possible to implement the connection structure 10 capable of preventing leakage of a fluid when the male connector 1 as a medical instrument is connected to the female connector 2 as a medical instrument.

The male connector 1 and the female connector 2 can form, for example, an infusion set. The infusion set includes, for example, the male connector 1, the female connector 2, an infusion container, a drip chamber, and a medical tube. The fluid is not particularly limited, and is, for example, a drug solution (an infusion solution, a nutrient, an anticancer agent, or the like), and a physiological salt solution.

Although the embodiment of the disclosure has been described above, the disclosure is not limited to the above-described embodiment, and the above-described embodiment can be variously modified without departing from the gist of the disclosure.

### Reference Signs List

1: male connector
2: female connector
2a: elastic valve body
2a1: slit
2b: movable body hooked portion
3: flow path pipe member
3a: opening
4: elastic opening and closing portion
4a: distal end surface
4b: slit
5: biasing portion
6: housing
6a: housing restriction portion
6a1: housing hooking portion
6b: housing base portion
6c: guide portion
6d: operation unit
7: movable body
7a: movable body restriction portion
7a1: movable body hooking portion
7a2: arm
7a3: protrusion
7a4: guided portion
7b: holding portion
7c: movable body base portion
8: housing hooked portion
9: inclined portion
10: connection structure
C1: male connector flow path
C2: female connector flow path
S: hollow

## Claims

1. A male connector connectable to a female connector having an elastic valve body, the male connector comprising:
a flow path pipe member that defines an axial direction and a radial direction and has an opening at a portion on an axial distal side;
an elastic opening and closing portion;
a biasing portion capable of biasing the elastic opening and closing portion toward the axial distal side;
a housing; and
a movable body that includes a movable body restriction portion and holds the elastic opening and closing portion, wherein
a form is changed from a first form in which the opening of the flow path pipe member is closed by the elastic opening and closing portion to a second form in which the opening of the flow path pipe member is opened by a portion of the flow path pipe member on the axial distal side protruding from the elastic opening and closing portion to the axial distal side by a connection operation in which the elastic opening and closing portion and the movable body move to an axial proximal side with respect to the flow path pipe member against a biasing force by the biasing portion by pressing the elastic valve body against a distal end surface of the elastic opening and closing portion,
the movable body restriction portion restricts movement of the female connector to the axial distal side with respect to the elastic opening and closing portion by moving inward in the radial direction due to a resistance force from the housing generated according to the connection operation, and
the housing includes a housing restriction portion that restricts the movement of the female connector to the axial distal side with respect to the flow path pipe member through the movable body restriction portion by restricting movement of the movable body to the axial distal side with respect to the flow path pipe member according to a change from the first form to the second form.

2. The male connector according to claim 1, wherein
the movable body restriction portion restricts the movement of the female connector to the axial distal side with respect to the elastic opening and closing portion by being hooked on the female connector.

3. The male connector according to claim 1, wherein
the housing restriction portion restricts the movement of the movable body to the axial distal side by being hooked on the movable body or the elastic opening and closing portion.

4. The male connector according to claim 1, wherein
the movable body includes a housing hooked portion on the axial proximal side with respect to the movable body restriction portion, and
the housing restriction portion includes a housing hooking portion to be hooked on the housing hooked portion.

5. The male connector according to claim 1, wherein
the movable body restriction portion includes an arm extending radially outward and toward the axial distal side, and a protrusion protruding inward in the radial direction from an end portion of the arm on the axial distal side.

6. The male connector according to claim 5, wherein
the protrusion of the movable body restriction portion is located on the axial distal side with respect to the distal end surface of the elastic opening and closing portion in the first form.

7. The male connector according to claim 1, wherein
the movable body includes a plurality of the movable body restriction portions arranged in a circumferential direction defined by the flow path pipe member.

8. The male connector according to claim 7, wherein
the movable body includes a pair of the movable body restriction portions facing each other in a predetermined radial direction and having an equal width in the circumferential direction, and another pair of the movable body restriction portions facing each other in another radial direction perpendicular to the predetermined radial direction and having an equal width in the circumferential direction.

9. The male connector according to claim 1, wherein
the elastic opening and closing portion and the biasing portion form an integrated elastic member.

10. The male connector according to claim 1, wherein
the male connector is configured as a medical instrument.

11. A connection structure comprising:
the male connector according to claim 1; and
the female connector.

12. The connection structure according to claim 11, wherein
the male connector and the female connector are each configured as a medical instrument.
